(19) Europäisches Patentamt
European Patent Office
Office européen des brevets

(11) Veröffentlichungsnummer: **0 034 743**
**B1**

# (12) EUROPÄISCHE PATENTSCHRIFT

(45) Veröffentlichungstag der Patentschrift: **30.05.84**

(51) Int. Cl.³: **C 07 D 413/04,** A 61 K 31/50 // (C07D413/04, 263/58, 237/04)

(21) Anmeldenummer: **81100890.3**

(22) Anmeldetag: **09.02.81**

## (54) Neue Benzoxazole, deren Herstellung und Verwendung als Arzneimittel.

(30) Priorität: **22.02.80 DE 3006671**

(43) Veröffentlichungstag der Anmeldung: **02.09.81 Patentblatt 81/35**

(45) Bekanntmachung des Hinweises auf die Patenterteilung: **30.05.84 Patentblatt 84/22**

(84) Benannte Vertragsstaaten: **AT BE CH DE FR GB IT LI LU NL SE**

(56) Entgegenhaltungen: **DE - A - 2 436 279**

(73) Patentinhaber: **Dr. Karl Thomae GmbH, Postfach 1755, D-7950 Biberach (Riss) (DE)**

(72) Erfinder: **Hauel, Norbert, Dr. Dipl.-Chem., Händelstrasse 12, D-7950 Biberach 1 (DE)**
Erfinder: **Heider, Joachim, Dr. Dipl.-Chem., Am Hang 3, D-7951 Warthausen 1 (DE)**
Erfinder: **Stein, Herbert, Dr. Dipl.-Chem., Mozartstrasse 3, D-7950 Biberach 1 (DE)**
Erfinder: **Austel, Volkhard, Dr. Dipl.-Chem., Kapellenweg 7, D-7950 Biberach 1 (DE)**
Erfinder: **Reiffen, Manfred, Dr. Dipl.-Chem., Amriswilstrasse 7, D-7950 Biberach 1 (DE)**
Erfinder: **Diederen, Willi, Dr., Haldenstrasse 1a, D-7950 Biberach 1 (DE)**
Erfinder: **Haarmann, Walter, Dr., Schlierholzweg 27, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der vorliegenden Erfindung sind neue Benzoxazole der allgemeinen Formel

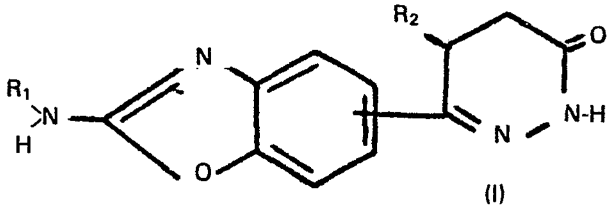

(I)

deren physiologisch verträgliche Säureadditionssalze mit anorganischen oder organischen Säuren und Verfahren zu ihrer Herstellung sowie diese Verbindungen enthaltende Arzneimittel.

Die Verbindungen der obigen allgemeinen Formel I weisen wertvolle pharmakologische Eigenschaften auf, insbesondere cardiovasculäre Wirkungen, nämlich cardiotonische, blutdrucksenkende und/oder antithrombotische.

In der obigen allgemeinen Formel I bedeutet

$R_1$ ein Wasserstoffatom, eine geradkettige oder verzweigte Alkylgruppe mit 1 bis 7 Kohlenstoffatomen, welche durch eine Alkoxygruppe, mit 1 bis 3 Kohlenstoffatomen substituiert sein kann, eine Cycloalkylgruppe mit 3 bis 7 Kohlenstoffatomen, eine durch eine Phenylgruppe, die durch ein oder zwei Alkoxygruppen mit 1 bis 3 Kohlenstoffatomen substituiert sein kann, substituierte Alkylgruppe mit 1 bis 3 Kohlenstoffatomen oder eine Phenylgruppe, welche durch eine Alkyl- oder Alkoxygruppe mit jeweils 1 bis 3 Kohlenstoffatomen, durch eine Trifluormethyl-, Nitro- oder Cyangruppe, durch ein Fluor-, Chlor- oder Bromatom substituiert sein kann, wobei zusätzlich eine der vorstehend genannten monosubstituierten Phenylgruppen durch ein oder zwei Alkylgruppen mit 1 bis 3 Kohlenstoffatomen oder durch eine Alkoxygruppe mit 1 bis 3 Kohlenstoffatomen oder durch ein Fluor-, Chlor- oder Bromatom substituiert sein kann, und

$R_2$ ein Wasserstoffatom oder eine Alkylgruppe mit 1 bis 3 Kohlenstoffatomen.

Für die bei der Definition der Reste $R_1$ und $R_2$ eingangs erwähnten Bedeutungen kommt somit für $R_1$ die Bedeutung des Wasserstoffatoms, der Methyl-, Äthyl-, Propyl-, Isopropyl-, n-Butyl-, Isobutyl-, tert.-Butyl-, n-Pentyl-, Isopentyl-, Neopentyl-, tert.-Pentyl-, hexyl-, Heptyl-, Methoxyäthyl-, Methoxypropyl-, Methoxybutyl-, Methoxypentyl-, Methoxyheptyl-, Äthoxyäthyl-, Propoxypropyl-, Cyclopropyl-, Cyclopentyl-, Cyclohexyl-, Cycloheptyl-, Phenyl-, Methylphenyl-, Dimethylphenyl-, Trimethylphenyl-, Äthylphenyl-, Propylphenyl-, Isopropylphenyl-, Methoxyphenyl-, Dimethoxyphenyl-, Isopropoxyphenyl-, Fluorphenyl-, Difluorphenyl-, Chlorphenyl-, Dichlorphenyl-, Bromphenyl-, Dibromphenyl-, Trifluormethyphenyl-, Nitrophenyl-, Cyanophenyl-, Methyl-fluorphenyl-, Methyl-chlorphenyl-, Methoxy-chlorphenyl-, Methoxy-bromphenyl-, Methoxy-cyanophenyl-, Methoxy-trifluormethylphenyl-, Methoxy-nitrophenyl-, Äthoxy-nitrophenyl-, Propoxy-nitrophenyl-, Benzyl-, 1-Phenyläthyl-, 3-Phenyläthyl-, Methoxybenzyl-, Dimethoxybenzyl-, 2-Dimethoxyphenyläthyl-, 2-Diäthoxyphenyläthyl-, 2-Dipropoxyphenyläthyl-, 2-(Methoxy-äthoxyphenyl)-äthyl- oder 3-Dimethoxy-phenylpropylgruppe und

für $R_2$ die des Wasserstoffatoms oder Methyl-, Äthyl-, Propyl- oder Isopropylgruppe in Betracht.

Bevorzugte Verbindungen der obigen allgemeinen Formel I sind diejenigen, in der

$R_1$ ein Wasserstoffatom, eine Alkylgruppe mit 1 bis 5 Kohlenstoffatomen, welche durch eine Methoxygruppe substituiert sein kann, eine Phenylgruppe, welche durch eine Methyl-, Methoxy-, Trifluormethyl-, Cyano- oder Nitrogruppe, durch ein Chlor- oder Bromatom substituiert sein kann, eine Dichlorphenyl-, Dibromphenyl-, Dimethoxyphenyl-, Methoxy-nitrophenyl-, Trimethylphenyl-, Cyclohexyl- oder Dimethoxyphenyläthylgruppe und

$R_2$ eine Methylgruppe bedeuten, insbesondere jedoch diejenigen, in der die Pyridazinongruppe in Position 5 des Benzoxazols steht, und deren physiologisch verträgliche Säureadditionssalze.

Besonders bevorzugte Verbindungen sind die Verbindungen der allgemeinen Formel

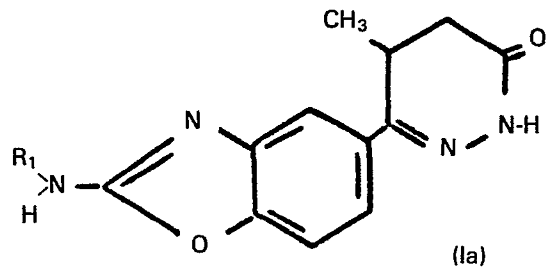

(Ia)

in der

$R_1$ eine Alkylgruppe mit 1 bis 5 Kohlenstoffatomen, eine Cyclohexylgruppe oder eine gegebenenfalls durch eine oder zwei Methoxygruppen substituierte Phenylgruppe darstellt, und deren physiologisch verträgliche Säureadditionssalze.

Erfindungsgemäss erhält man die neuen Verbindungen der allgemeinen Formel I nach folgenden Verfahren:

a) Cyclisierung eines Thioharnstoffs der allgemeinen Formel

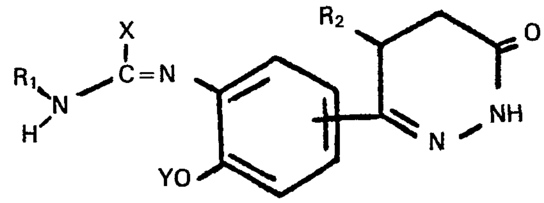

(II)

in der

$R_1$ und $R_2$ wie eingangs definiert sind und

X eine Mercapto-, Alkylmercapto-, Arylmercapto- oder Aralkylmercaptogruppe und

Y ein Wasserstoffatom oder eine Acylgruppe bedeuten.

Die Umsetzung wird zweckmässigerweise in einem Lösungsmittel wie Tetrahydrofuran, Dioxan,

Benzol, Toluol oder Dimethyläthylenglykol vorzugsweise in Gegenwart eines Kondensationsmittels wie N,N'-Dicyclohexyl-carbodiimid, Carbonyl-diimidazol, p-Toluolsulfonsäure, Phosphoroxychlorid, Thionylchlorid, Salzsäure, Schwefelsäure, Phosphorsäure oder Polyphosphorsäure bei Temperaturen zwischen 25 und 200°C, vorzugsweise jedoch bei der Siedetemperatur des Reaktionsgemisches, z.B. bei Temperaturen zwischen 50 und 150°C, durchgeführt. Die Umsetzung kann jedoch auch in der Schmelze durchgeführt werden.

b) Umsetzunbg einer Carbonsäure der allgemeinen Formel

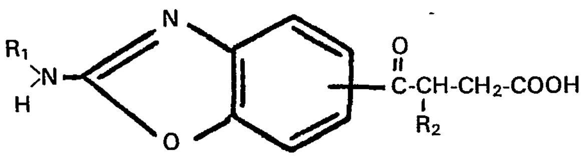

(III)

in der

$R_1$ und $R_2$ wie eingangs definiert sind, oder deren Ester, Thioester, Amide oder Halogenide mit Hydrazin.

Die Umsetzung wird zweckmässigerweise in einem Lösungsmittel wie Äthanol, Isopropanol, Eisessig, Propionsäure und/oder in einem Überschuss von Hydrazin bzw. Hydrazin-hydrat bei Temperaturen zwischen 50 und 200°C, vorzugsweise bei der Siedetemperatur des Reaktionsgemisches, z.B. bei Temperaturen zwischen 75 und 150°C, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durchgeführt werden.

Eine gemäss den Verfahren a) oder b) erhaltene Verbindung der allgemeinen Formel I, in der $R_1$ ein Wasserstoffatom darstellt, kann anschliessend mittels Alkylierung in eine entsprechende Verbindung der allgemeinen Formel I, in der $R_1$ mit Ausnahme des Wasserstoffatoms und der Phenylreste wie eingangs definiert ist, übergeführt werden.

Die nachträgliche Alkylierung wird mit einem entsprechenden Alkylierungsmittel wie einem Phenylalkylhalogenid, Alkylhalogenid, Dialkylsulfat oder Trialkyloxonium-tetrafluorborat, z.B. mit Benzylchlorid, Phenyläthylbromid, Methyljodid, Dimethylsulfat, Diäthylsulfat oder Äthylbromid, zweckmässigerweise in einem Lösungsmittel wie Methanol, Äthanol, Methanol/Wasser oder Dioxan gegebenenfalls in Gegenwart einer Base wie Natriumcarbonat, Natriumbicarbonat, Natriumhydroxid, Natriummethylat oder Kaliumcarbonat bei Temperaturen bis zur Siedetemperatur des verwendeten Lösungsmittels durchgeführt.

Ferner können die erhaltenen Verbindungen der allgemeinen Formel I aufgrund ihres optisch aktiven Kohlenstoffatoms in Position 5 des Pyridazinon-Ringes in ihre optisch aktiven Antipoden mittels Racematspaltung aufgetrennt werden. Die Racematspaltung wird zweckmässigerweise durch fraktionierte Kristallisation der entsprechenden Salze mit optisch aktiven Säuren, wie Weinsäure, Dibenzoylweinsäure, Äpfelsäure, Camphersäure oder Camphersulfonsäure, oder durch Chromatographie an optisch aktiven Adsorbenten durchgeführt.

Desweiteren können die erhaltenen Verbindungen der allgemeinen Formel I in ihre physiologisch verträglichen Salze mit anorganischen oder organischen Säuren übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III erhält man nach literaturbekannten Methoden. So erhält man beispielsweise eine Verbindung der allgemeinen Formel II durch Umsetzung eines entsprechenden 3-(Nitro-hydroxybenzoyl)-buttersäureesters mit Hydrazin, anschliessende Reduktion der Nitrogruppe und anschliessende Umsetzung der so erhaltenen Verbindung mit einem entsprechenden Thioisocyanat.

Eine Verbindung der allgemeinen Formel III erhält man durch Umsetzung einer Verbindung der allgemeinen Formel

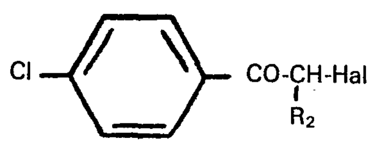

(IV)

mit Malonester. Die so erhaltene Verbindung wird anschliessend verseift, decarboxyliert, nitriert, das Chloratom durch eine Hydroxygruppe ersetzt, die Nitrogruppe reduziert und die so erhaltene Aminoverbindung nach Umsetzung mit einem entsprechenden Thioisocyanat zu dem gewünschten Benzoxazol cyclisiert.

Die neuen Verbindungen der allgemeinen Formel I und deren optisch aktiven Antipoden sowie deren physiologisch verträglichen Säureadditionssalze mit anorganischen oder organischen Säuren weisen, wie bereits eingangs erwähnt, wertvolle pharmakologische Eigenschaften auf, insbesondere cardiovasculäre Wirkungen, nämlich eine cardiotonische, blutdrucksenkende und/oder antithrombotische.

Beispielsweise wurden die Verbindungen

A = 5-Methyl-6-[2'-ethylamino-benzoxazol-5'-yl]-4,5-dihydro-3(2H)pyridazinon,

B = 5-Methyl-6-[2'-cyclohexylamino-benzoxazol-5'-yl]-4,5-dihydro-3(2H)pyridazinon,

C = 5-Methyl-6-[2'-n-butylamino-benzoxazol-5'-yl]-4,5-dihydro-3(2H)pyridazinon und

D = 5-Methyl-6-[2'-(4-methoxyphenylamino)-benzoxazol-5'-yl]-4,5-dihydro-3(2H)pyridazinon

auf ihre biologischen Eigenschaften wie folgt untersucht:

1. *Bestimmung der Thrombozytenaggregation nach Born und Cross [J. Physiol.* 170, *397 (1964)]:*

Die Thrombozytenaggregation wurde in plättchenreichem Plasma gesunder Versuchspersonen gemessen. Hierbei wurde der Verlauf der Abnahme der optischen Dichte nach Zugabe von handelsüblichem Collagen der Firma Sigma, St. Louis/USA, welches 1 mg Collagen-Fibrillen pro ml enthält, photometrisch gemessen und registriert. Aus dem Neigungswinkel der Dichtekurve wurde auf die Aggregationsgeschwindigkeit (Vmax) geschlossen. Der Punkt der Kurve, bei dem die grösste Lichtdurchlässigkeit vorlag, diente zur Berechnung der «optical density» (O.D.). Zur maximalen Aggregationsauslösung werden ca., 0,01 ml der Collagenlösung zu 1 ml plättchenreichem Plasma gegeben.

Die nachfolgende Tabelle enthält den gefundenen Wert:

*Tabelle I*

| Verbindung | $EC_{50}$ in $\mu$Mol/l |
|---|---|
| A | 1,4 |

2. *Bestimmung der blutdrucksenkenden und positiv inotropen Wirkung an der narkotisierten Katze:*

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Milliar PC-350 A) der Druck in der linken Herzkammer gemessen. Daraus wurde der Kontraktilitätsparameter $dp/dt_{max}$ mittels eines Analogdifferenzierers gewonnen.

Die zu untersuchenden Substanzen wurden in eine Vena femoralis injiziert. Als Lösungsmittel diente physiologische Kochsalz-Lösung oder Polydiol 200. Jede Substanz wurde an mindestens 3 Katzen geprüft, Dosis 0,1 oder 2,0 mg/kg i.v. Die Wirkungsdauer der untersuchten Substanzen beträgt jeweils mindestens 45 Minuten.

Die nachfolgende Tabelle enthält die Mittelwerte:

| Substanz | Dosis mg/kg i.v. | Blutdruckänderung in mmHg | Zunahme von dp/dt in % |
|---|---|---|---|
| A | 0,1 | —40/33 | +79 |
| B | 0,1 | —32/32 | +39 |
| C | 0,1 | —27/27 | +62 |
| D | 2,0 | —32/42 | +92 |

3. *Akute Toxizität:*

Die akute Toxizität der zu untersuchenden Substanzen wurde an weissen Mäusen nach oraler Gabe einer einmaligen Dosis orientierend bestimmt (Beobachtungszeit: 14 Tage):

*Tabelle III*

| Substanz | Akute Toxizität mg/kg p.o. |
|---|---|
| A | > 300 (2 von 6 Tieren gestorben) |
| B | > 300 (0 von 6 Tieren gestorben) |
| C | > 300 (1 von 6 Tieren gestorben) |
| D | > 300 (0 von 6 Tieren gestorben) |

Aufgrund ihrer pharmakologischen Eigenschaften eigenen sich die erfindungsgemäss hergestellten Verbindungen der allgemeinen Formel I und deren optisch aktive Antipoden sowie deren physiologisch verträgliche Säureadditionssalze mit anorganischen oder organischen Säuren zur Behandlung der chronischen Herzinsuffizienz oder der Angina Pectoris und/oder zur Prophylaxe artierieller Thromboembolien und arterieller Verschlusskrankheiten.

Hierzu lassen sich die neuen Benzoxazole, gegebenenfalls in Kombination mit anderen Wirksubstanzen, in die üblichen pharmazeutischen Anwendungsformen wie Tabletten, Dragées, Pulver, Suspensionen, Suppositorien oder Ampullen einarbeiten. Die Einzeldosis beträgt hierbei beim Erwachsenen 1-4 × täglich bei intravenöser Applikation 10-50 mg, vorzugsweise 20 bis 40 mg, und bei oraler Applikation 50-150 mg, vorzugsweise 75 bis 100 mg.

Die nachfolgenden Beispiele sollen die Erfindung näher erläutern:

*Beispiel I*

*N-Ethyl-N'-[2-hydroxy-5-(5-methyl-4,5-dihydro-3(2H)pyridazinon-6-yl)phenyl]thioharnstoff*

10,8 g (50 mMol) 2-Amino-4-(5-methyl-4,5-dihydro-3(2H)pyridazinon-6-yl)phenol werden in 250 ml Tetrahydrofuran suspendiert, mit 25 ml (0,29 Mol) Ethylisothiocyanat versetzt und die Mischung 3,5 Stunden lang unter Rühren zum Rückfluss erhitzt. Dabei bildet sich schon nach kurzer Zeit eine klare Lösung. Nach beendeter Reaktionszeit wird das Lösungsmittel abgedampft und der gelbe ölige Rückstand durch Zusatz von Ether kristallisiert. Das ausgefallene Produkt wird abgesaugt, mit Ether gewaschen und bei Raumtemperatur getrocknet.

Ausbeute: 13,0 g (91,5% der Theorie)
Schmelzpunkt: 137-139°C
$C_{14}H_{18}N_4O_2S$ (306,4)

| | | | | |
|---|---|---|---|---|
| Ber.: | C 54,88 | H 5,92 | N 18,29 | S 10,47 |
| Gef.: | C 55,10 | H 6,10 | N 17,90 | S 10,30 |

*Beispiel II*

*2-Ethylamino-5-(3-methyl-4-oxo-buttersäure-4-yl)-benzoxazol*

3,24 g (10,0 mMol) N-Ethyl-N'-[2-hydroxy-5-(3-methyl-4-oxo-buttersäure-4-yl)phenyl]thioharnstoff werden in 150 ml Tetrahydrofuran gelöst, mit 4,17 g (20 mMol) N,N'-Dicyclohexylcarbodiimid versetzt und 5 Stunden lang unter Rückfluss erhitzt. Nach beendeter Reaktionszeit wird zur Hälfte eingeengt, nochmals kurz aufgekocht und zur Kristallisation gekühlt. Das entstandene Produkt wird abgesaugt, aus Tetrahydrofuran umkristallisiert und bei Raumtemperatur getrocknet.

Ausbeute: 0,71 g (24,3 % der Theorie)
Schmelzpunkt: 160-162°C

*Beispiel 1*

*5-Methyl-6-[2'-methylamino-benzoxazol-5'-yl]-4,5-dihydro-3(2H)pyridazinon*

3,36 g (11,5 mMol) N-Methyl-N'-[2-hydroxy-5-(5-methyl-4,5-dihydro-3(2H)pyridazinon-6-yl)phenyl]thioharnstoff werden in 150 ml Tetrahydrofuran gelöst, mit 3,13 g (15 mMol) N,N'-Dicyclohexylcarbodiimid versetzt und 5 Stunden lang am Rückfluss erhitzt. Nach beendeter Reaktionszeit wird zur Hälfte eingeengt, nochmals kurz aufgekocht und zur Kristallisation gekühlt. Das entstandene Produkt wird abgesaugt, aus Methanol umkristallisiert und bei Raumtemperatur getrocknet.

Ausbeute: 0,84 g (28,3 % der Theorie)
Schmelzpunkt: 241-242°C
$C_{13}H_{14}N_4O_2$ (258,3)
Ber.: C 60,46 H 5,46 N 21,69
Gef.: C 60,28 H 5,61 N 21,50

*Beispiel 2*

*5-Methyl-6-[2'-n-butylamino-benzoxazol-5'-yl]-4,5-dihydro-3(2H)pyridazinon*

Hergestellt analog Beispiel 1 aus N-n-Butyl-N'-[2-hydroxy-5-(5-methyl-4,5-dihydro-3(2H)pyridazinon-6-yl)phenyl]thioharnstoff.

Ausbeute: 39,7 % der Theorie
Schmelzpunkt: 186-188°C
$C_{16}H_{20}N_4O_2$ (300,4)
Ber.: C 63,98 H 6,71 N 18,65
Gef.: C 63,99 H 6,96 N 18,54

*Beispiel 3*

*5-Methyl-6-[2'-isopropylamino-benzoxazol-5'-yl]-4,5-dihydro-3(2H)pyridazinon*

Hergestellt analog Beispiel 1 aus N-Isopropyl-N'-[2-hydroxy-5-(5-methyl-4,5-dihydro-3(2H)pyridazinon-6-yl)phenyl]thioharnstoff.

Ausbeute: 52 9 der Theorie
Schmelzpunkt: 239-241°C
$C_{15}H_{18}N_4O_2$ (286,34)
Ber.: C 69,92 H 6,34 N 19,57
Gef.: C 62,65 H 6,33 N 19,25

*Beispiel 4*

*5-Methyl-6-[2'-cyclohexylamino-benzoxazol-5'-yl]-4,5-dihydro-3(2H)pyridazinon*

Hergestellt analog Beispiel 1 aus N-Cyclohexyl-N'-[2-hydroxy-5-(5-methyl-4,5-dihydro-3(2H)-pyridazinon-6-yl)phenyl]thioharnstoff.

Ausbeute: 59,1 % der Theorie
Schmelzpunkt: 235-237°C
$C_{18}H_{22}N_4O_2$ (326,4)
Ber.: C 66,24 H 6,79 N 17,16
Gef.: C 66,00 H 6,90 N 16,99

*Beispiel 5*

*5-Methyl-6-[2'-(3,4-dimethoxyphenethylamino)-benzoxazol-5'-yl]-4,5-dihydro-3(2H)pyridazinon*

Hergestellt analog Beispiel 1 aus N-3,4-Dimethoxyphenethyl-N'-[2-hydroxy-5-(5-methyl-4,5-dihydro-3(2H)pyridazinon-6-yl)phenyl]thioharnstoff.

Ausbeute: 48,2 % der Theorie
Schmelzpunkt: 70-72°C
$C_{22}H_{24}N_4O_4 \times HCl$ (444,93)
Ber.: C 59,39 H 5,87 N 12,59 Cl 7,99
Gef.: C 59,40 H 5,69 N 12,89 Cl 8,00

*Beispiel 6*

*5-Methyl-6-[2'-phenylamino-benzoxazol-5'-yl]-4,5-dihydro-3(2H)pyridazinon*

5 ml Hydrazinhydrat (99%ig) werden unter Rühren und Eiskühlung bei ca. 20°C portionsweise zu 20 ml Eisessig gegeben. Anschliessend werden 6,5 g (20,0 mMol) 2-Phenylamino-5-(3-methyl-4-oxo-buttersäure-4-yl)benzoxazol zugesetzt und die so erhaltene Suspension am Rückfluss erhitzt. Nach 1 Stunde Reaktionszeit wird auf Raumtemperatur abgekühlt, mit Wasser verdünnt, das ausgefallene Produkt abgesaugt, mit Wasser gewaschen und bei 80°C getrocknet.

Ausbeute: 2,80 g (43,7 % der Theorie)
Schmelzpunkt: 214-217°C
$C_{18}H_{16}N_4O_2$ (320,34)
Ber.: C 67,49 H 5,03 N 17,49
Gef.: C 67,00 H 5,32 N 17,12

*Beispiel 7*

*5-Methyl-6-[2'-(2-chloranilino)-benzoxazol-5'-yl]-4,5-dihydro-3(2H)pyridazinon*

Hergestellt analog Beispiel 1 aus N-2-Chlorphenyl-N'-[2-hydroxy-5-(5-methyl-4,5-dihydro-3(2H)pyridazinon-6-yl)phenyl]thioharnstoff.

Ausbeute: 33,9 der Theorie
Schmelzpunkt: 193-195°C
$C_{18}H_{15}N_4O_2$ (354,8)
Ber.: C 60,93 H 4,26 N 15,79 Cl 9,99
Gef.: C 60,90 H 4,25 N 15,93 Cl 10,20

*Beispiel 8*

*5-Methyl-6-[2'-(4-trifluormethylphenylamino)-benzoxazol-5'-yl]-4,5-dihydro-3(2H)pyridazinon*

Hergestellt analog Beispiel 1 aus N-4-Trifluormethylphenyl-N'-[2-hydroxy-5-(5-methyl-4,5-dihydro-3(2H)pyridazinon-6-yl)-phenyl]thioharnstoff.

Ausbeute: 57,3 g % der Theorie
Schmelzpunkt: 249-251°C
$C_{19}H_{15}N_4O_2F_3$ (388,36)
Ber.: C 58,76 H 3,89 N 14,42
Gef.: C 59,00 H 4,13 N 14,31

*Beispiel 9*

*5-Methyl-6-[2'-(3,4-dichlorphenylamino)benzoxazol-5'-yl]-4,5-dihydro-3(2H)pyridazinon*

Hergestellt analog Beispiel 1 aus N-3,4-Dichlorphenyl-N'-[2-hydroxy-5-(5-methyl-4,5-dihydro-3(2H)-oyridazinon-6-yl)phenyl]thioharnstoff.

Ausbeute: 73,5 % der Theorie
Schmelzpunkt: 253-256°C
$C_{18}H_{14}N_4O_2Cl_2$ (389,3)
Ber.: C 55,54 H 3,63 Cl 18,22
Gef.: C 55,40 H 3,83 Cl 18,14

*Beispiel 10*

*5-Methyl-6-[2'-(4-bromphenylamino)-benzoxazol-5'-yl]-4,5-dihydro-3(2H)pyridazinon*

Hergestellt analog Beispiel 1 aus N-4-Bromphenyl-N'-[2-hydroxy-5-(5-methyl-4,5-dihydro-3(2H)-

pyridazinon-6-yl)phenyl]thioharnstoff.
Ausbeute: 65,4 % der Theorie
Schmelzpunkt: 243-245°C
$C_{18}H_{15}N_4N_2Br$ (390,27)

| | | | | |
|---|---|---|---|---|
| Ber.: | C 55,40 | H 3,87 | N 14,36 | Br 20,48 |
| Gef.: | C 55,00 | H 4,05 | N 13,95 | Br 20,76 |

*Beispiel 11*

*5-Methyl-6-[2'-(4-methoxyphenylamino)benzoxazol-5'-yl]-4,5-dihydro-3(2H)pyridazinon*

Hergestellt analog Beispiel 1 aus N-4-Methoxyphenyl-N'-[2-hydroxy-5-(5-methyl-4,5-dihydro-3(2H)pyridazinon-6-yl)phenyl]thioharnstoff.
Ausbeute: 40,3 % der Theorie
Schmelzpunkt: 220 - 222°C
$C_{19}H_{18}N_4O_3$ (350,4)

| | | | |
|---|---|---|---|
| Ber.: | C 65,13 | H 5,18 | N 15,99 |
| Gef.: | C 65,30 | H 5,20 | N 15,83 |

*Beispiel 12*

*5-Methyl-6-[2'-(2-methoxy-4-nitrophenylamino)-benzoxazol-5'-yl]-4,5-dihydro-3(2H)pyridazinon*

Hergestellt analog Beispiel 1 aus N-2-Methoxy-4-nitrophenyl-N'-[2-hydroxy-5-(5-methyl-4,5-dihydro-3(2H)pyridazinon-6-yl)phenyl]thioharnstoff.
Ausbeute: 65,6 % der Theorie
Schmelzpunkt: 223-225°C
$C_{19}H_{17}N_5O_5$ (395,4)

| | | | |
|---|---|---|---|
| Ber.: | C 57,82 | H 4,33 | N 17,71 |
| Gef.: | C 58,27 | H 4,25 | N 18,17 |

*Beispiel 13*

*5-Methyl-6-[2'-(4-methylphenylamino)benzoxazol-5'-yl]-4,5-dihydro-3(2H)pyridazinon*

Hergestellt analog Beispiel 1 aus N-4-Methylphenyl-N'-[2-hydroxy-5-(5-methyl-4,5-dihydro-3(2H)-pyridazinon-6-yl)phenyl]thioharnstoff.
Ausbeute: 52,1 % der Theorie
Schmelzpunkt: 273-275°C
$C_{19}H_{18}N_4O_2$ (334,39)

| | | | |
|---|---|---|---|
| Ber.: | C 68,25 | H 5,43 | N 16,76 |
| Gef.: | C 68,10 | H 5,59 | N 16,88 |

*Beispiel 14*

*5-Methyl-6-[2'-(2,4,6-trimethylphenylamino)benzoxazol-5'-yl]-4,5-dihydro-3(2H)pyridazinon*

Hergestellt analog Beispiel 1 aus N-2,4,6-Trimethylphenyl-N'-[2-hydroxy-5-(5-methyl-4,5-dihydro-3(2H)pyridazinon-6-yl)phenyl]thioharnstoff.
Ausbeute: 76,4 % der Theorie
Schmelzpunkt: 225-228°C
$C_{21}H_{22}N_4O_2$ (362,4)

| | | | |
|---|---|---|---|
| Ber.: | C 69,59 | H 6,12 | N 15,46 |
| Gef.: | C 69,79 | H 6,37 | N 15,65 |

*Beispiel 15*

*5-Methyl-6-[2'-(4-cyanophenylamino)benzoxazol-5'-yl]-4,5-dihydro-3(2H)pyridazinon*

Hergestellt analog Beispiel 1 aus N-4-Cyanophenyl-N'-[2-hydroxy-5-(5-methyl-4,5-dihydro-3(2H)-pyridazinon-6-yl)phenyl]thioharnstoff.
Ausbeute: 59,7 % der Theorie
Schmelzpunkt: 318-320°C
$C_{19}H_{15}N_5O_2$ (345,37)

| | | | |
|---|---|---|---|
| Ber.: | C 66,08 | H 4,38 | N 20,28 |
| Gef.: | C 66,14 | H 4,58 | N 20,03 |

*Beispiel 16*

*5-Methyl-6-[2'-n-pentylamino-benzoxazol-5'-yl]-4,5-dihydro-3(2H)pyridazinon*

Hergestellt analog Beispiel 1 aus N-n-Pentyl-N'-[2-hydroxy-5-(5-methyl-4,5-dihydro-3(2H)pyridazinon-6-yl)phenyl]thioharnstoff.
Ausbeute: 52,3 % der Theorie
Schmelzpunkt: 154-155°C
$C_{17}H_{22}N_4O_2$ (314,4)

| | | | |
|---|---|---|---|
| Ber.: | C 64,95 | H 7,05 | N 17,82 |
| Gef.: | C 65,26 | H 7,12 | N 17,88 |

*Beispiel 17*

*5-Methyl-6-[2'-ethylamino-benzoxazol-5'-yl]-4,5-dihydro-3(2H)pyridazinon*

Hergestellt analog Beispiel 1 aus N-Ethyl-N'-[2-hydroxy-5-(5-methyl-4,5-dihydro-3(2H)pyridazinon-6-yl)phenyl]thioharnstoff.
Ausbeute: 71,9 % der Theorie
Schmelzpunkt: 244-246°C
$C_{14}H_{16}N_4O_2$ (272,3)

| | | | |
|---|---|---|---|
| Ber.: | C 61,75 | H 5,92 | N 20,57 |
| Gef.: | C 61,95 | H 5,98 | N 20,85 |

*Beispiel 18*

*5-Methyl-6-[2'-(3-methoxypropylamino)benzoxazol-5'-yl]-4,5-dihydro-3(2H)pyridazinon*

Hergestellt analog Beispiel 1 aus N-3-Methoxypropyl-N'-[2-hydroxy-5-(5-methyl-4,5-dihydro-3(2H)pyridazinon-6-yl)phenyl]thioharnstoff.
Ausbeute: 55,5 % der Theorie
Schmelzpunkt: 153-154°C
$C_{16}H_{20}N_4O_3$ (316,4)

| | | | |
|---|---|---|---|
| Ber.: | C 60,75 | H 6,37 | N 17,71 |
| Gef.: | C 61,00 | H 6,40 | N 17,56 |

*Beispiel 19*

*5-Methyl-6-[2'-amino-benzoxazol-5'-yl]-4,5-dihydro-3(2H)pyridazinon*

Hergestellt analog Beispiel 1 aus N-[2-Hydroxy-5-(5-methyl-4,5-dihydro-3(2H)pyridazinon-6-yl)phenyl]thioharnstoff.
Ausbeute: 42,1 % der Theorie
Schmelzpunkt: 285-287°C.

*Beispiel A*

*Tabletten zu 100 mg 5-Methyl-6-[2'-ethylamino-benzoxazol-5'-yl]-4,5-dihydro-3(2H)pyridazinon*

Zusammensetzung:
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Wirkstoff und Milchzucker mit wässriger Lösung des Polyvinylpyrrolidons gleichmässig befeuchten.

Feuchtsiebung: 1,5 mm
Trocknen: Umlufttrockenschrank 50°C
Trockensieben: 1 mm

Dem Granulat die restlichen Hilfsstoffe zumischen und Endmischung zu Tabletten verpressen.

Tablettengewicht: 175 mg
Stempel: 8 mm ∅

*Beispiel B*

*Dragées zu 50 mg 5-Methyl-6-[2'-ethylamino--benzoxazol-5'-yl]-4,5-dihydro-3(2H)pyridazinon*

1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wässriger Lösung der löslichen Stärke gleichmässig befeuchten.

Feuchtsiebung: 1,0 mm
Trockensiebung: 1,0 mm
Trocknung: 50°C im Umlufttrockenschrank

Granulat und restliche Hilfsstoffe mischen und zu Kernen verpressen.

Kerngewicht: 80 mg
Stempel: 6 mm
Wölbungsradius: 5 mm

Die fertigen Kerne werden auf übliche Weise mit einem Zuckerüberzug im Dragierkessel versehen.

Dragéegewicht: 120 mg

*Beispiel C*

*Suppositorien zu 75 mg 5-Methyl-6-[2'-ethylamino--benzoxazol-5'-yl]-4,5-dihydro-3(2H)pyridazinon*

1 Zäpfchen enthält:

| | |
|---|---|
| Wirksubstanz | 75,0 mg |
| Zäpfchenmasse (z.B. Witepsol H 19 und Witepsol W 45) | 1625,0 mg |
| | 1700,0 mg |

*Herstellungsverfahren:*

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

*Beispiel D*

*Ampullen zu 25 mg 5-Methyl-6-[2'-ethylamino--benzoxazol-5'-yl]-4,5-dihydro-3(2H)pyridazinon*

Zusammensetzung:

1 Ampulle enthält:

| | |
|---|---|
| Wirksubstanz | 25,0 mg |
| Natriumchlorid | 45,0 mg |
| Polyäthylenglykol 600 | 1,0 ml |
| Lösungsvermittler (z.B. hydroxyäthyliertes hydriertes Rizinusöl) | 0,5 ml |
| Wasser für Injektionszwecke ad | 5,0 ml |

*Herstellungsverfahren:*

In einem geeigneten volumengeeichten Gefäss wird der Wirkstoff in einem Gemisch aus Polyäthylenglykol, Lösungsvermittler und ca. der Hälfte des Wassers unter Rühren gelöst. Nach vollständiger Lösung wird das Isotonans Natriumchlorid zugesetzt und mit Wasser auf das Nennvolumen aufgefüllt.

*Beispiel E*

*Suspension mit 75 mg 5-Methyl-6-[2'-ethylamino--benzoxazol-5'-yl]-4,5-dihydro-3(2H)pyridazinon pro 5 ml*

100 ml Suspension enthalten:

| | |
|---|---|
| Wirksubstanz | 1,5 g |
| Carboxymethylcellulose | 0,1 g |
| p-Hydroxybenzoesäuremethylester | 0,05 g |
| p-Hydroxybenzoesäurepropylester | 0,03 g |
| Rohrzucker | 10,0 g |
| Glycerin | 5,0 g |
| Sorbitlösung 70% | 20,0 g |
| Aroma | 0,3 g |
| Wasser dest. ad | 100,0 ml |

*Herstellungsverfahren:*

Dest. Wasser wird auf 70°C erhitzt. Hierin wird unter Rühren p-Hydroxybenzoesäureethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

**Patentansprüche für die Vertragsstaaten:**
BE, CH, DE, FR, GB, IT, LI, LU, NL, SE

1. Benzoxazole der allgemeinen Formel

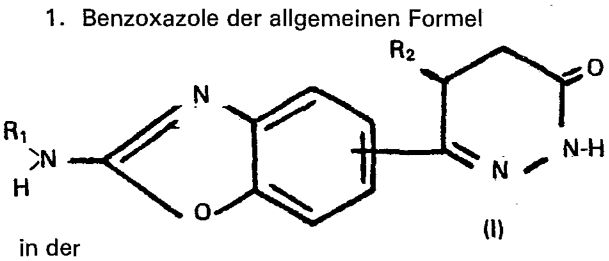

(I)

in der

$R_1$ ein Wasserstoffatom, eine geradkettige oder verzweigte Alkylgruppe mit 1 bis 7 Kohlenstoffatomen, welche durch eine Alkoxygruppe, mit 1 bis 3 Kohlenstoffatomen substituiert sein kann, eine Cycloalkylgruppe mit 3 bis 7 Kohlenstoffatomen, eine durch eine Phenylgruppe, die durch ein oder zwei Alkoxygruppen mit 1 bis 3 Kohlenstoffatomen substituiert sein kann, substituierte Alkylgruppe mit 1 bis 3 Kohlenstoffatomen oder eine Phenylgruppe, welche durch eine Alkyl- oder Alkoxygruppe mit jeweils 1 bis 3 Kohlenstoffatomen, durch eine Trifluormethyl-, Nitro- oder

Cyangruppe. durch ein Fluor-, Chlor-, oder Bromatom substituiert sein kann, wobei zusätzlich eine der vorstehend genannten monosubstituierten Phenylgruppen durch ein oder zwei Alkylgruppen mit 1 bis 3 Kohlenstoffatomen oder durch eine Alkoxygruppe mit 1 bis 3 Kohlenstoffatomen oder durch ein Fluor-, Chlor- oder Bromatom substituiert sein kann, und

$R_2$ ein Wasserstoffatom oder eine Alkylgruppe mit 1 bis 3 Kohlenstoffatomen bedeuten, und deren optisch aktiven Antipoden sowie deren physiologisch verträgliche Säureadditionssalze mit anorganischen oder organischen Säuren.

2. Benzoxazole der allgemeinen Formel

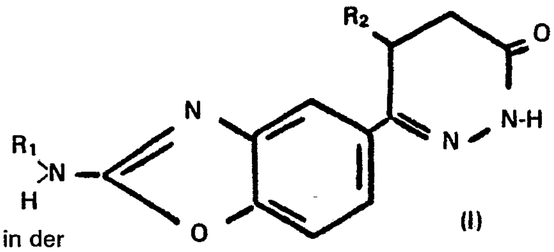

(I)

in der

$R_1$ und $R_2$ wie im Anspruch 1 definiert sind, und deren optisch aktiven Antipoden sowie deren physiologisch verträgliche Säureadditionssalze mit anorganischen oder organischen Säuren.

3. Benzoxazole der allgemeinen Formel I gemäss Anspruch 2, in der

$R_1$ ein Wasserstoffatom, eine Alkylgruppe mit 1 bis 5 Kohlenstoffatomen, welche durch eine Methoxygruppe substituiert sein kann, eine Phenylgruppe, welche durch eine Methyl-, Methoxy-, Trifluormethyl-, Cyano- oder Nitrogruppe, durch ein Chlor- oder Bromatom substituiert sein kann, eine Dichlorphenyl-, Dibromphenyl-, Dimethoxyphenyl-, Methoxy-nitrophenyl-, Trimethylphenyl-, Cyclohexyl- oder Dimethoxyphenyläthylgruppe und

$R_2$ eine Methylgruppe bedeuten, und derenm optisch aktiven Antipoden sowie deren physiologisch verträgliche Säureadditionssalze mit anorganischen Säuren.

4. Benzoxazole der allgemeinen Formel I gemäss Anspruch 2, in der

$R_1$ eine Alkylgruppe mit 1 bis 5 Kohlenstoffatomen, die Cyclohexylgruppe oder eine gegebenenfalls durch eine oder zwei Methoxygruppen substituierte Phenylgruppe und

$R_2$ die Methylgruppe bedeuten, und deren optisch aktiven Antipoden sowie deren physiologisch verträgliche Säureadditionssalze mit anorganischen oder organischen Säuren.

5. 5-Methyl-6-[2'-ethylamino-benzoxazol-5'-yl]-4,5-dihydro-3(2H)pyridazinon und dessen optisch aktiven Antipoden sowie deren Säureadditionssalze.

6. 5-Methyl-6-[2'-cyclohexylamino-benzoxazol-5'-yl]-4,5-dihydro-3(2H)pyridazinon und dessen optisch aktiven Antipoden sowie deren Säureadditionssalze.

7. 5-Methyl-6-[2'-n-butylamino-benzoxazol-5'-yl]-4,5-dihydro-3(2H)pyridazinon und dessen optisch aktiven Antipoden sowie deren Säureadditionssalze.

8. Arzneimittel, enthaltend eine Verbindung gemäss den Ansprüchen 1-7 neben einem oder mehreren inerten Trägerstoffen und/oder Verdünnungsmitteln.

9. Verwendung einer Verbindung gemäss den Ansprüchen 1-7 zur Herstellung eines Arzneimittels zur Bekämpfung von cardiovaskulären Erkrankungen auf nichtchemischem Wege.

10. Verfahren zur Herstellung von neuen Benzoxazolen gemäss den Ansprüchen 1-7 und von deren optisch aktiven Antipoden sowie von deren physiologisch verträglichen Säureadditionssalzen mit anorganischen oder anorganischen Säuren, dadurch gekennzeichnet, dass

a) ein Thioharnstoff der allgemeinen Formel

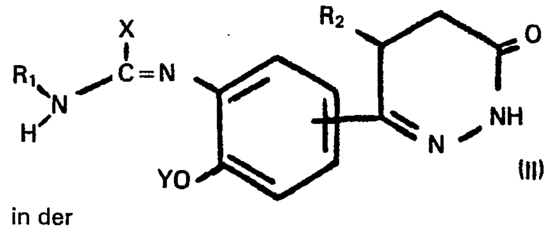

(II)

in der

$R_1$ und $R_2$ wie eingangs definiert sind,

X eine Mercapto-, Alkylmercapto-, Arylmercapto- oder Aralkylmercaptogruppe und

Y ein Wasserstoffatom oder einen Acylrest bedeuten, ringgeschlossen wird oder

b) eine Carbonsäure der allgemeinen Formel

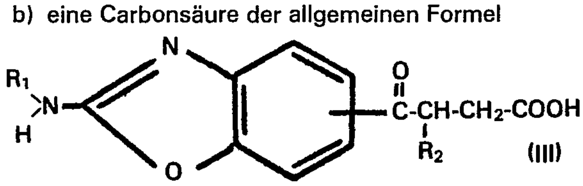

(III)

in der

$R_1$ und $R_2$ wie eingangs definiert sind, oder deren Amide, Ester, Thioester oder Halogenide mit Hydrazin umgesetzt wird

und gewünschtenfalls anschliessend eine gemäss den Verfahren a) oder b) erhaltene Verbindung der allgemeinen Formel I, in der $R_1$ ein Wasserstoffatom darstellt, mittels Alkylierung in eine entsprechende Verbindung der allgemeinen Formel I, in der $R_1$ mit Ausnahme des Wasserstoffatoms und der eingangs erwähnten Phenylreste wie eingangs definiert ist, und/oder ein erhaltenes Racemat der allgemeinen Formel I mittels Racematspaltung in ihre optisch aktiven Antipoden aufgespalten wird

und/oder eine erhaltene Verbindung der allgemeinen Formel I in ihre physiologisch verträglichen Säureadditionssalze mit anorganischen oder organischen Säuren übergeführt wird.

**Patentansprüche für den Vertragsstaat:** AT

1. Verfahren zur Herstellung von Benzoxazolen der allgemeinen Formel

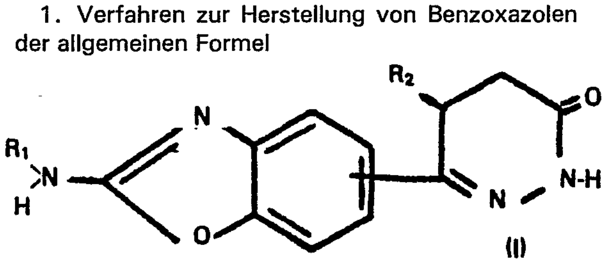

(I)

in der

$R_1$ ein Wasserstoffatom, eine geradkettige oder verzweigte Alkylgruppe mit 1 bis 7 Kohlenstoffatomen, welche durch eine Alkoxygruppe, mit 1 bis 3 Kohlenstoffatomen substituiert sein kann, eine Cycloalkylgruppe mit 3 bis 7 Kohlenstoffatomen, eine durch eine Phenylgruppe, die durch ein oder zwei Alkoxygruppen mit 1 bis 3 Kohlenstoffatomen substituiert sein kann, substituierte Alkylgruppe mit 1 bis 3 Kohlenstoffatomen oder eine Phenylgruppe, welche durch eine Alkyl- oder Alkoxygruppe mit jeweils 1 bis 3 Kohlenstoffatomen, durch eine Trifluormethyl-, Nitro- oder Cyangruppe, durch ein Fluor-, Chlor- oder Bromatom substituiert sein kann, wobei zusätzlich eine der vorstehend genannten monosubstituierten Phenylgruppen durch ein oder zwei Alkylgruppen mit 1 bis 3 Kohlenstoffatomen oder durch eine Alkoxygruppe mit 1 bis 3 Kohlenstoffatomen oder durch ein Fluor-, Chlor- oder Bromatom substituiert sein kann, und

$R_2$ ein Wasserstoffatom oder eine Alkylgruppe mit 1 bis 3 Kohlenstoffatomen bedeuten, und von deren optisch aktiven Antipoden sowie von deren physiologisch verträglichen Säureadditionssalzen mit anorganischen oder organischen Säuren, dadurch gekennzeichnet, dass

a) ein Thioharnstoff der allgemeinen Formel

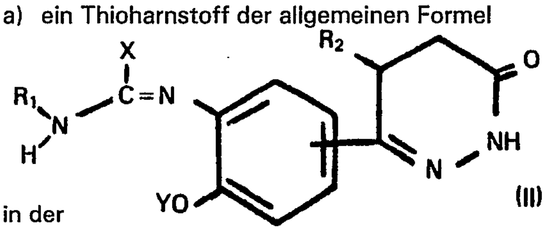

(II)

in der

$R_1$ und $R_2$ wie eingangs definiert sind,

X eine Mercapto-, Alkylmercapto-, Arylmercapto- oder Aralkylmercaptogruppe und

Y ein Wasserstoffatom oder einen Acylrest bedeuten, ringgeschlossen wird oder

b) eine Carbonsäure der allgemeinen Formel

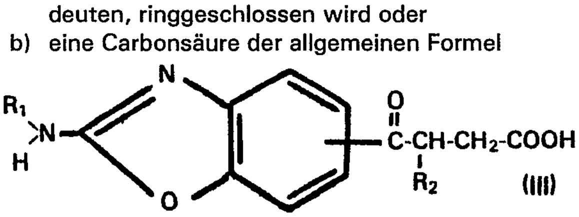

(III)

in der

$R_1$ und $R_2$ wie eingangs definiert sind, oder deren Amide, Ester, Thioester oder Halogenide mit Hydrazin umgesetzt wird

und gewünschtenfalls anschliessend eine gemäss den Verfahren a) oder b) erhaltene Verbindung der allgemeinen Formel I, in der $R_1$ ein Wasserstoffatom darstellt, mittels Alkylierung in eine entsprechende Verbindung der allgemeinen Formel I, in der $R_1$ mit Ausnahme des Wasserstoffatoms und der eingangs erwähnten Phenylreste wie eingangs definiert ist,

und/oder ein erhaltenes Racemat der allgemeinen Formel I mittels Racematspaltung in ihre optisch aktiven Antipoden aufgespalten wird

und/oder eine erhaltene Verbindung der allgemeinen Formel I in ihre physiologisch verträglichen Säureadditionssalze mit anorganischen oder organischen Säuren übergeführt wird.

2. Verfahren gemäss Anspruch 1a, dadurch gekennzeichnet, dass die Umsetzung bei Temperaturen zwischen 25 und 300°C durchgeführt wird.

3. Verfahren gemäss den Ansprüchen 1a und 2, dadurch gekennzeichnet, dass die Umsetzung in einem Lösungsmittel durchgeführt wird.

4. Verfahren gemäss den Ansprüchen 1a, 2 und 3, dadurch gekennzeichnet, dass die Cyclisierung in Gegenwart eines Kondensationsmittels durchgeführt wird.

5. Verfahren gemäss Anspruch 1b, dadurch gekennzeichnet, dass die Umsetzung bei Temperaturen zwischen 0 und 200°C durchgeführt wird.

6. Verfahren gemäss Anspruch 5, dadurch gekennzeichnet, dass die Umsetzung bei Temperaturen zwischen 20 und 150°C durchgeführt wird.

7. Verfahren gemäss den Ansprüchen 1b und 6, dadurch gekennzeichnet, dass die Umsetzung in einem Lösungsmittel durchgeführt wird.

8. Verfahren gemäss den Ansprüchen 1b, 6 und 7, dadurch gekennzeichnet, dass die Umsetzung in Gegenwart eines sauren Kondensationsmittels durchgeführt wird.

9. Verfahren zur Herstellung einer galenischen Zubereitung, dadurch gekennzeichnet, dass eine Verbindung gemäss Anspruch 1 zusammen mit üblichen inerten Trägerstoffen und/oder Verdünnungsmitteln verarbeitet wird.

**Claims for the Contracting States:**
BE, CH, DE, FR, GB, IT, LI, LU, NL

1. Benzoxazoles of general formula

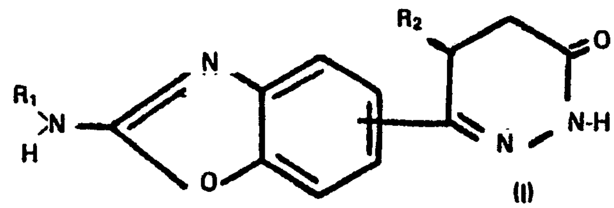

(I)

in which

$R_1$ represents a hydrogen atom; a straight or branched alkyl group with 1 to 7 carbon atoms which can be substituted by an alkoxy group with 1 to 3 carbon atoms; a cycloalkyl group with 3 to 7 carbon atoms; an alkyl group with 1 to 3 carbon atoms substituted by a phenyl group which can be substituted by one or two alkoxy groups with 1 to 3 carbon atoms; or a phenyl group which can be substituted by an alkyl or alkoxy group each with 1 to 3 carbon atoms, by a trifluormethyl, nitro or cyano group or by a fluorine, chlorine or bromine atom; and in addition one of the mono-substituted phenyl groups mentioned above can be substituted by one or two alkyl groups with 1 to 3 carbon atoms or by an alkoxy group with 1 to 3 carbon atoms or by a fluorine, chlorine or bromine atom; and

$R_2$ represents a hydrogen atom or an alkyl group with 1 to 3 carbon atoms;

and their optically active antipodes as well as their physiologically compatible acid addition salts with inorganic or organic acids.

2. Benzoxazoles of general formula

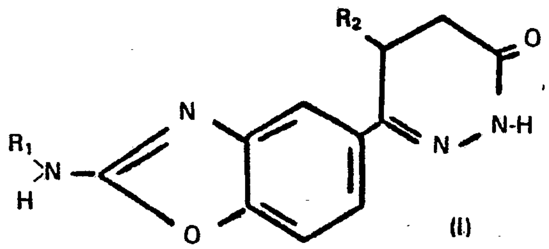

(I)

in which
$R_1$ and $R_2$ are as defined in claim 1, and their optically active antipodes as well as their physiologically compatible acid addition salts with inorganic or organic acids.

3. Benzoxazoles of general formula I according to claim 2 in which

$R_1$ represents a hydrogen atom; an alkyl group with 1 to 5 carbon atoms which can be substituted by a methoxy group; a phenyl group which can be substituted by a methyl, methoxy, trifluoromethyl, cyano or nitro group or by a chlorine or bromine atom; a dichlorophenyl, dibromophenyl, dimethoxyphenyl, methoxynitrophenyl, trimethylphenyl, cyclohexyl or dimethoxyphenylethyl group; and

$R_2$ represents a methyl group;

and their optically active antipodes as well as their physiologically compatible acid addition salts with inorganic or organic acids.

4. Benzoxazoles of general formula I according to claim 2 in which

$R_1$ represents an alkyl group with 1 to 5 carbon atoms; the cyclohexyl group or a phenyl group optionally substituted by one or two methoxy groups; and

$R_2$ represents the methyl group;

and their optically active antipodes as well as their physiologically compatible acid addition salts with inorganic or organic acids.

5. 5-Methyl-6-[2'-ethylamino-benzoxazol-5'-yl]-4,5-dihydro-3(2H)pyridazinone and its optically active antipodes as well as their acid addition salts.

6. 5-Methyl-6-[2'-cyclohexylamino-benzoxazol-5'-yl]-4,5-dihydro-3(2H)pyridazinone and its optically active antipodes as well as their acid addition salts.

7. 5-Methyl-6-[2'-n-butylamino-benzoxazol-5'-yl]-4,5-dihydro-3(2H)pyridazinone and its optically active antipodes as well as their acid addition salts.

8. Pharmaceutical preparations containing a compound according to claims 1 to 7 in addition to one or more inert carriers and/or diluents.

9. Use of a compound according to claims 1 to 7 for producing a pharmaceutical preparation for controlling cardiovascular diseases in a non-chemical way.

10. Process for producing new benzoxazoles according to claims 1 to 7 and their optically active antipodes as well as their physiologically compatible acid addition salts with inorganic or organic acids, characterised in that

a) a thiourea of general formula

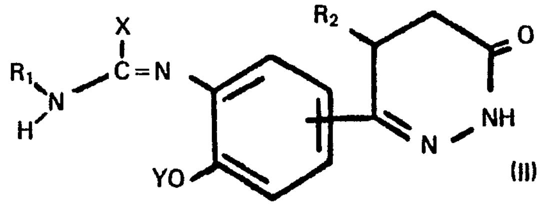

(II)

in which

$R_1$ and $R_2$ are as defined in the introduction,

X represents a mercapto, alkylmercapto, arylmercapto or aralkylmercapto group, and

Y represents a hydrogen atom or an acyl radical, is cyclised; or

b) a carboxylic acid of general formula

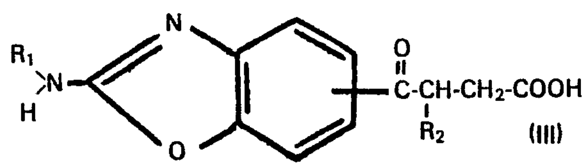

(III)

in which

$R_1$ and $R_2$ are as defined in the introduction, or its amides, esters, thioesters or halides, is reacted with hydrazine, and if desired a compound of general formula I, in which $R_1$ represents a hydrogen atom, obtained according to processes a) or b) is subsequently converted by means of alkylation into a corresponding compound of general formula I, in which $R_1$ is defined as in the introduction with the exception of the hydrogen atom and the phenyl radicals men-tioned in the introduction, and/or a racemate ob-tained, of general formula I, is separated by means of racemate separation into its optically active antipodes, and/or a compound obtained, of general formula I, is converted into its physiologically compatible acid addition salts with inorganic or organic acids.

**Claims for the Contracting State:** AT

1. Process for producing benzoxazoles of general formula

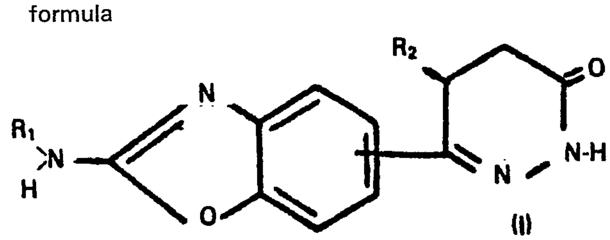

(I)

in which

$R_1$ represents a hydrogen atom; a straight or branched alkyl group with 1 to 7 carbon atoms which can be substituted by an alkoxy group with 1 to 3 carbon atoms; a cycloalkyl group with 3 to 7 carbon atoms; an alkyl group with 1 to 3 carbon atoms substituted by a phenyl group which can be substituted by one or two alkoxy groups with 1 to 3 carbon atoms; or a phenyl group which can be substituted by an alkyl or alkoxy group each with 1 to 3 carbon atoms, by a trifluormethyl, nitro or cyano group or by a fluorine, chlorine or bromine atom; and additionally one of the mono-substituted phenyl groups mentioned above can be substituted by one or two alkyl groups with 1 to 3 carbon atoms or by an alkoxy group with 1 to 3 carbon atoms or by a fluorine, chlorine or bromine atom; and

$R_2$ represents a hydrogen atom or an alkyl group with 1 to 3 carbon atoms;
and their optically active antipodes as well as their physiologically compatible acid addition salts with inorganic or organic acids characterised in that

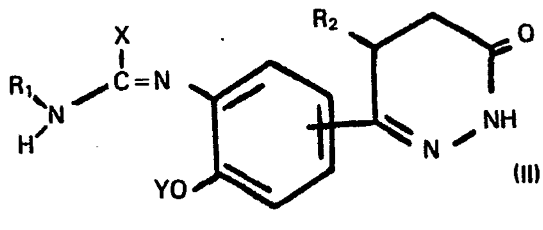

(II)

in which

$R_1$ and $R_2$ are as defined in the introduction,

X represents a mercapto, alkylmercapto, arylmercapto or aralkylmercapto group, and

Y represents a hydrogen atom or an acyl radical, is cyclised; or

b) a carboxylic acid of general formula

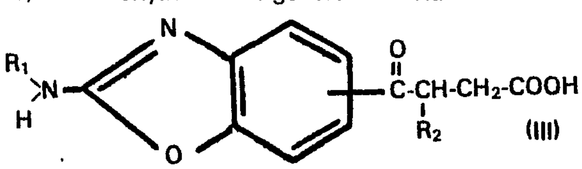

(III)

in which

$R_1$ and $R_2$ are as defined in the introduction, or its amides, esters, thioester or halides, is reacted with hydrazine;

and if desired a compound of general formula I, in which $R_1$ represents a hydrogen atom, obtained according to processes a) or b) is subsequently converted by means of alkylation into a corresponding compound of general formula I, in which $R_1$ is defined as in the introduction with the exception of the hydrogen atom and the phenyl radicals mentioned in the introduction, and/or a racemate obtained, of general formula I, is separated by means of racemate separation into its optically active antipodes, and/or a compound obtained, of general formula I, is converted into its physiologically compatible acid addition salts with inorganic or organic acids.

2. Process according to claim 1a, characterised in that the reaction is carried out at temperatures of between 25 and 300°C.

3. Process according to claims 1a und 2, characterised in that the reaction is carried out in a solvent.

4. Process according to claims 1a, 2 and 3, characterised in that cyclising is carried out in the presence of a condensation agent.

5. Process according to claim 1b, characterised in that the reaction is carried out at temperatures of between 0 and 200°C.

6. Process according to claim 5, characterised in that the reaction is carried out at temperatures of between 20 and 150°C.

7. Process according to claims 1b and 6, characterised in that the reaction is carried out in a solvent.

8. Process according to claims 1b, 6 and 7, characterised in that the reaction is carried out in the presence of an acid condensation agent.

9. Process for producing a galenic preparation, characterised in that a compound according to claim 1 is processed together with conventional inert carriers and/or diluents.

**Revendications pour les Etats Contractants:**
BE, CH, DE, FR, GB, IT, LI, LU, NL, SE

1. Benzoxazoles des formule générale:

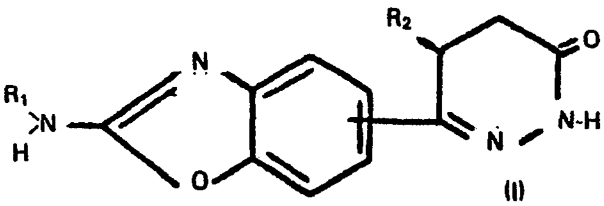

(I)

dans laquelle:

$R_1$ représente un atome d'hydrogène, un groupe alcoyle rectiligne ou ramifié avec 1 à 7 atomes de carbone, lequel peut être substitué par un groupe alcoxy avec 1 à 3 atomes de carbone, un groupe cycloalcoyle avec 3 à 7 atomes de carbone, un groupe alcoyle avec 1 à 3 atomes de carbone substitué par un groupe phényle qui peut être substitué par un ou deux groupes alcoxy avec 1 à 3 atomes de carbone ou représente un groupe phényle, lequel peut être substitué par un groupe alcoyle ou alcoxy avec à chaque fois 1 à 3 atomes de carbone, par un groupe trifluorométhyle, nitro ou cyano, par un atome de fluor, de chlore ou de brome, l'un des groupes phényle monosubstitués mentionnés précédemment pouvant être en outre substitué par un ou deux groupes alcoyle avec 1 à 3 atomes de carbone ou par un groupe alcoxy avec 1 à 3 atomes de carbone ou par un atome de fluor, de chlore ou de brome, et

$R_2$ représente un atome d'hydrogène ou un groupe alcoyle avec 1 à 3 atomes de carbone, et leurs antipodes optiquement actifs ainsi que leurs sels d'addition d'acides physiologiquement supportables avec des acides minéraux ou organiques.

2. Benzoxazoles de formule générale:

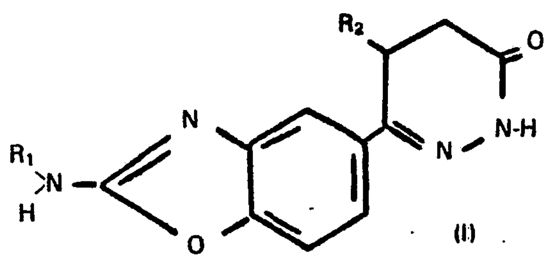

(I)

dans laquelle:

$R_1$ et $R_2$ sont définis comme dans la revendication 1, et leurs antipodes optiquement actifs ainsi que leurs sels d'addition d'acides physiologiquement supportables avec des acides minéraux ou organiques.

3. Benzoxazoles de formule générale I selon la revendication 2, dans laquelle:

$R_1$ représente un atome d'hydrogène, un groupe alcoyle avec 1 à 5 atomes de carbone, lequel peut être substitué par un groupe méthoxy, ou représente un groupe phényle, lequel peut être substitué par un groupe méthyle, méthoxy, trifluorométhyle, cyano ou nitro, par un atome de chlore ou de brome, ou représente un groupe dichlorophényle, dibromophényle, diméthoxyphényle, méthoxynitrophényle, triméthylphényle, cyclohexyle ou diméthoxyphényléthyle et

$R_2$ représente un groupe méthyle, et leurs antipodes optiquement actifs ainsi que leurs sels d'addition d'acides physiologiquement supportables avec des acides minéraux ou organiques.

4. Benzoxazoles de formule générale I selon la revendication 2, dans laquelle:

$R_1$ représente un groupe alcoyle avec 1 à 5 atomes de carbone, le groupe cyclohexyle ou un groupe phényle éventuellement substitué par un ou deux groupes méthoxy et

$R_2$ représente le groupe méthyle, et leurs antipodes optiquement actifs ainsi que leurs sels d'addition d'acides physiologiquement supportables avec des acides minéraux ou organiques.

5. La 5-méthyl-6-[2'-éthylamino-benzoxazol-5'-yl]-4,5-dihydro-3(2H)pyridazinone et ses antipodes optiquement actifs ainsi que leurs sels d'addition d'acides.

6. La 5-méthyl-6-[2'-cyclohexylamino-benzoxazol-5'-yl]-4,5-dihydro-3(2H)pyridazinone et ses antipodes optiquement actifs ainsi que leurs sels d'addition d'acides.

7. La 5-méthyl-6-[2'-n-butylamino-benzoxazol-5'-yl]-4,5-dihydro-3(2H)pyridazinone et ses antipodes optiquement actifs ainsi que leurs sels d'addition d'acides.

8. Médicament contenant un composé selon les revendications 1 à 7 conjointement à un ou plusieurs excipients et/ou diluants inertes.

9. Utilisation d'un composé selon les revendications 1 à 7 pour la préparation d'un médicament pour lutter contre les maladies cardiovasculaires par voie non chimique.

10. Procédé pour la préparation de nouveaux benzoxazoles selon les revendications 1 à 7 et de leurs antipodes optiquement actifs ainsi que de leurs sels d'addition d'acides physiologiquement supportables avec des acides minéraux ou organiques, caractérisé en ce que:

a) une thiourée de formule générale:

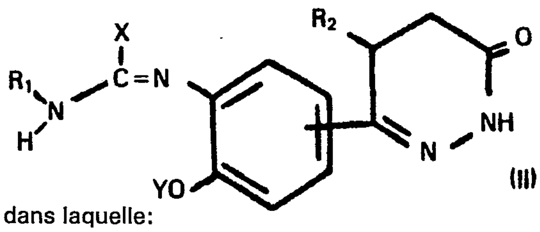

(II)

dans laquelle:

$R_1$ et $R_2$ sont définis comme au début,

X représente un groupe mercapto, alcoylmercapto, arylmercapto ou aralcoylmercapto et

Y représente un atome d'hydrogène ou un radical acyle, subit une fermeture de cycle ou

b) un acide carboxylique de formule générale:

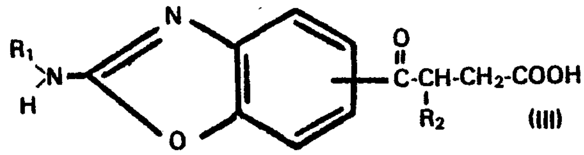

(III)

dans laquelle:

$R_1$ et $R_2$ sont définis comme au début, ou son amide, ester, thioester ou halogénure réagit avec l'hydrazine,

et si on le désire, un composé de formule générale I dans laquelle $R_1$ représente un atome d'hydrogène, obtenu selon le procédé a) ou b), est transformé ensuite au moyen d'une alcoylation en un composé correspondant de formule générale I dans laquelle $R_1$ est défini comme au début à l'exception de l'atome d'hydrogène et des radicaux phényle mentionnés au début,

et/ou un racémate obtenu, de formule générale I, est clivé au moyen d'un clivage de racémate en ses antipodes optiquement actifs

et/ou un composé obtenu de formule générale I est transformé en ses sels d'addition d'acides physiologiquement supportables avec des acides minéraux ou organiques.

**Revendications pour l'Etat Contractant:** AT

1. Procédé pour la préparation de benzoxazoles de formule générale:

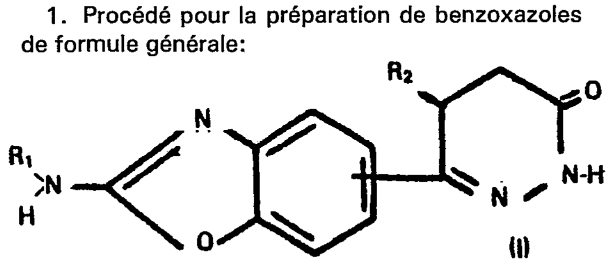

(I)

dans laquelle:

$R_1$ représente un atome d'hydrogène, un groupe alcoyle rectiligne ou ramifié avec 1 à 7 atomes de carbone, lequel peut être substitué par un groupe alcoxy avec 1 à 3 atomes de carbone, un groupe cycloalcoyle avec 3 à 7 atomes de carbone, un groupe alcoyle avec 1 à 3 atomes de carbone substitué par un groupe phényle qui peut être substitué par un ou deux groupes alcoxy avec 1 à 3 atomes de carbone ou représente un groupe phényle, lequel peut être substitué par un groupe alcoyle ou alcoxy avec à chaque fois 1 à 3 atomes de carbone, par un groupe trifluorométhyle, nitro ou cyano, par un atome de fluor, de chlore ou de brome, l'un des groupes phényle monosubstitués mentionnés précédemment pouvant être en outre substitué par un ou deux groupes alcoyle avec 1 à 3 atomes de carbone ou par un groupe alcoxy avec 1 à 3 atomes de carbone ou par un atome de fluor, de chlore ou de brome, et

$R_2$ représente un atome d'hydrogène ou un groupe alcoyle avec 1 à 3 atomes de carbone, et de leurs antipodes optiquement actifs ainsi que de leurs sels d'addition d'acides physiologiquement supportables avec des acides minéraux ou organiques, caractérisé en ce que:

a) une thiourée de formule générale:

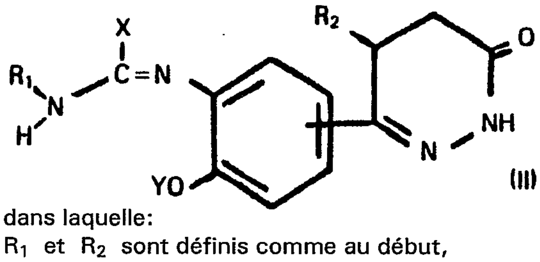

(II)

dans laquelle:

$R_1$ et $R_2$ sont définis comme au début,

X représente un groupe mercapto, alcoylmercapto, arylmercapto ou aralcoylmercapto et

Y représente un atome d'hydrogène ou un radical acyle, subit une fermeture de cycle ou

b) un acide carboxylique de formule générale:

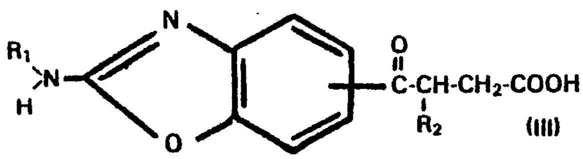

(III)

dans laquelle:

$R_1$ et $R_2$ sont définis comme au début, ou son amide, ester, thioester ou halogénure réagit avec l'hydrazine,

et éventuellement ensuite un composé de formule générale I dans laquelle $R_1$ représente un atome d'hydrogène, obtenu selon le procédé a) ou b), est transformé au moyen d'une alcoylation en un composé correspondant de formule générale I dans laquelle $R_1$ est défini comme au début à l'exception de l'atome d'hydrogène et des radicaux phényle mentionnés au début,

et/ou un racémate obtenu, de formule générale I, est clivé au moyen d'un clivage de racémate en ses antipodes optiquement actifs

et/ou un composé obtenu de formule générale I est transformé en ses sels d'addition d'acides physiologiquement supportables avec des acides minéraux ou organiques.

2. Procédé selon la revendication 1a, caractérisé en ce que la réaction est effectuée à des températures entre 25 et 300°C.

3. Procédé selon les revendications 1a et 2, caractérisé en ce que la réaction est effectuée dans un solvant.

4. Procédé selon les revendications 1a, 2 et 3, caractérisé en ce que la cyclisation est effectuée en présence d'un agent de condensation.

5. Procédé selon la revendication 1b, caractérisé en ce que la réaction est effectuée à des températures entre 0 et 200°C.

6. Procédé selon la revendication 5, caractérisé en ce que la réaction est effectuée à des températures entre 20 et 150°C.

7. Procédé selon les revendications 1b et 6, caractérisé en ce que la réaction est effectuée dans un solvant.

8. Procédé selon les revendications 1b, 6 et 7, caractérisé en ce que la réaction est effectuée en présence d'un agent de condensation acide.

9. Procédé pour la fabrication d'une préparation galénique, caractérisé en ce qu'on traite un composé selon la revendication 1 ensemble avec des excipients et/ou diluants inertes habituels.